# EUROPEAN PATENT APPLICATION

(11) **EP 4 548 885 A1**
(43) Date of publication of application: **07.05.2025**
(21) Application number: 24211057.5
(22) Date of filing: 06.11.2024
(51) Int. Cl.: A61F 5/14

(54) **DISPOSABLE INSERT FOR THE TREATMENT OF PLANTAR SKIN LESIONS AND A KIT COMPRISING A PLURALITY OF SUCH DISPOSABLE INSERTS**

(30) Priority: 06.11.2023 IT 202300023226
(71) Applicant: Podarte S.r.l., 31044 Montebelluna (TV) (IT)
(72) Inventor: BURATTO, Camillo, 31044 Montebelluna (TV) (IT)
(74) Representative: Dragotti & Associati S.R.L.

(57) **Abstract**

The present invention relates to a disposable insert (10) for the treatment of plantar skin lesions (U), comprising a body (12) made of a polymer material and having a variable thickness (t) along its longitudinal axis (T). The body (12) comprises an upper surface (14), configured to support a given portion of the foot (F) of a user, and an opposite lower surface (16).

In accordance with the invention, the body (12) comprises a concavity (18) configured to be arranged facing, when the disposable insert (10) is being used, a plantar skin lesion (U) of the user's foot (F), so as to prevent said plantar lesion (U) from coming into contact with the body (12) of the insert (10) or with a support surface.

The invention also relates to a kit comprising a plurality of such disposable inserts (10).

## Description

The present invention relates to a disposable insert for the treatment of plantar skin lesions. More specifically, the present invention relates to a disposable insert for the treatment of plantar ulcers, such as diabetic foot ulcers, where "diabetic foot" is understood as meaning a chronic complication of diabetes mellitus, which causes anatomical/functional alterations of the foot and heel.

The present invention also relates to a kit comprising a plurality of such disposable inserts.

It is known that, among the most common complications of diabetes, there is the formation of plantar ulcers which significantly affect the quality of life of the patient.

Moreover, if not suitably treated, the ulcers may worsen to the point where amputation of the foot may be required.

In the present state of the art various treatments for curing diabetic foot ulcers are known.

These treatments principally aim to lighten the pressure in the area affected by the ulcer ("offloading") and are accompanied by debridement and topical treatments.

The international guidelines classify foot ulcers as follows:
- digital (toe) ulcers
- plantar ulcers
- midfoot ulcers
- heel ulcers

The standard treatment for achieving offloading of the area affected by the ulcer consists of a cast or resin boot, referred to by the technical term TCC (total contact cast). This boot may also be made of plastic.

However, in view of their complexity, boots of this type are used to treat less than 5% of ulcers.

Other instruments for achieving offloading are leg or heel braces (walkers). However, such braces are invasive and costly.

More commonly used, therefore, are so-called "heeling shoes", which are less costly and are designed to immobilize and relieve the pressure on the foot, but which generally require further personalized adaptation, such as the provision of suitable insoles.

The most common way of offloading plantar ulcers consists in providing an insert with a thickness of about 10 mm, made of wool felt.

Such an insert, in fact, is obtained by manually cutting it to the required size from a larger-size sheet. Moreover, it is required to form, again manually, a through-hole in the zone of the insert intended to face the ulcer, so as to prevent the ulcer from coming into direct contact with the felt.

This low-cost and practical solution, however, has various drawbacks. Firstly, cutting of the felt and making the hole in the insert are operations which are time-consuming and require a certain manual skill.

In any case, since they are operations which are performed manually, they are unable to ensure a high degree of precision and therefore there is the risk that the ulcer may in any case come into direct contact with the ground or partially with the felt, thus making the insert ineffective.

Felts tend, moreover, to flatten with use and under the weight of the patient, thus reducing offloading effect.

Moreover, the insert has a uniform thickness. It is therefore ineffective with regard to lightening the very heavy load pressures acting on the foot insole in the zones adjacent to the ulcer itself. There is therefore the risk that the ulcer may spread to the surrounding tissues, worsening the patient's condition.

Another way of offloading the ulcer consists in providing a flat insole, prepared by a doctor or specialized operator and provided with a hole in the zone intended to be arranged facing the ulcer.

This type of insole offers undoubted advantages compared to wool felt inserts, since it is less prone to collapse, but, in order to be effective, it requires good manual skill in order for the offloading to be achieved in the correct manner.

A third way consists in providing, by means of casting, an insole made to size and suitably shaped so as to match the shape of the patient's foot and provided with a suitable offloading discharge in the region of the foot sole affected by the ulcer.

This solution, which is able to treat in an optimum manner the patient's foot, has however a high cost and therefore is not particularly widely used.

The object of the present invention is therefore to overcome at least partially the drawbacks mentioned above in relation to the known devices used for offloading plantar ulcers.

In particular, a task of the present invention is to provide a disposable insert for the treatment of plantar skin lesions, which is both practical and easy to use.

Furthermore, a task of the present invention is to provide a disposable insert for the treatment of plantar skin lesions which may be easily produced and which has low production costs.

The aforementioned object and tasks are achieved by a disposable insert for the treatment of plantar skin lesions according to Claim 1 and by a kit comprising a plurality of disposable inserts according to Claim 18.

The characteristic features and further advantages of the invention will emerge from the description, provided hereinbelow, of a number of examples of embodiment, provided by way of a non-limiting example, with reference to the accompanying drawings in which:
- Figure 1 shows a perspective view of a first embodiment of an insert according to the present invention;
- Figure 2 shows a view, from above, of the insert according to Figure 1;
- Figure 3 shows a view, from behind, of the insert according to Figure 1;
- Figures 4 and 5 are two side views, from opposite sides, of the insert according to Figure 1;
- Figure 6 shows a perspective view of a second embodiment of an insert according to the present invention;
- Figure 7 shows a view, from above, of the insert according to Figure 6;
- Figure 8 shows a side view of the insert according to Figure 6;
- Figure 9 shows a perspective view of a third embodiment of an insert according to the present invention;
- Figure 10 shows a view, from above, of the insert according to Figure 9;
- Figure 11 shows a view, from behind, of the insert according to Figure 9;
- Figure 12 shows a side view of the insert according to Figure 9;
- Figure 13 shows a perspective view of a fourth embodiment of an insert according to the invention;
- Figure 14 shows a view, from above, of the insert according to Figure 13;
- Figure 15 shows a first side view of the insert according to Figure 13;
- Figure 16 shows a cross-sectional view along the plane indicated by XVI-XVI in Figure 15;
- Figure 17 shows a view, from behind, of the insert according to Figure 13;
- Figure 18 shows a second side view of the insert according to Figure 13;
- Figure 19 shows in schematic form a view, from below, of the insert according to Figure 1 applied to a left foot;
- Figure 20 shows a cross-sectional view along the plane indicated by XX-XX in Figure 19;
- Figure 21 shows in schematic form a view, from below, of the insert according to Figure 6 applied to a left foot;
- Figure 22 shows in schematic form a view, from below, of the insert according to Figure 9 applied to a foot;
- Figure 23 shows in schematic form a view, from below, of the insert according to Figure 13 applied to a foot;
- Figure 24 shows in schematic form a side view of Figure 23;
- Figure 25 shows in schematic form a cross-sectional view along the plane indicated by XXV-XXV in Figure 24;
- Figure 26 shows in a schematic form a side view of a further embodiment of the insert according to the invention applied to a foot;
- Figures 27 and 28 are figures similar to Figures 19 and 21, but which show the insert applied to a right foot.

The present invention relates to a disposable insert 10 for the treatment of plantar skin lesions U such as, for example, diabetic foot ulcers. In particular, the present invention relates to a disposable insert 10 suitable for offloading diabetic foot ulcers, i.e. for allowing patients to rest their foot on a support surface without their weight acting on the lesion zone.

Below, "front" will be used to identify the part of the insert, or its single components, configured to be positioned relatively closer to the toe end of the foot, while "rear" will be used to indicate the part of the insert, or its single components, configured to be positioned relatively closer to the heel. Similarly, "upper" will refer to the part of the insert, or its single components, configured to be arranged relatively closer to the user's foot, while "lower" will be used to indicate the part of the insert, or its single components, configured to be positioned relatively further away from the foot.

The terms "medial" and "lateral" will be understood, in a known manner, as being in relation to a hypothetical sagittal plane of the user of the insert, i.e. relative to the plane which extends from the head to the feet of the user, and in particular "medial" refers to the part of the insert configured to be positioned, during use, closer to the sagittal plane, while "lateral" refers to the part of the insert configured to be positioned, during use, further away from the sagittal plane.

"Longitudinal axis" of the insert will be understood as meaning the axis which extends from the rear part towards the front part of the said insert. Said longitudinal axis in the attached figures is indicated by the arrow T. Finally, "disposable inserts" are understood as meaning inserts intended to be thrown away after use.

With reference to the attached figures, the disposable insert 10 according to the invention comprises a body 12 made of a polymer material and having a thickness t variable along its longitudinal axis T. Advantageously the thickness t is between 7 and 12 mm. Preferably said thickness t is about 10 mm.

Said body 12 comprises an upper surface 14, configured to support a given portion of a foot F of the user, and an opposite lower surface 16.

In accordance with the invention, said body 12 comprises a concavity 18 configured to be arranged facing, when the insert 10 is being used, a plantar skin lesion U of the user's foot F, so as to prevent said lesion U from coming into contact with the body 12 of the insert 10 or with a support surface, for example the ground or a shoe portion, so as to ensure offloading of the lesion U.

As already mentioned, the body 12 of the lesion 12 is made of a polymer material. Advantageously, the polymer material is a closed-cell material. This type of material does not allow bacteria and fungi to adhere to it and, since it ensures a low residual permanent deformation after compression, i.e. a low "compression set", it prevents the insert 10 from collapsing. Preferably, the body 12 of the insert 10 has a hardness measured on the Shore A scale of between 20 and 50.

Advantageously, the body 12 of the insert 10 has a density of between 150 and 450 kg/m³.

The combination of hardness and density values mentioned above prevent the insert 10 from being deformed by the weight of the user. In this way, the insert 10 ensures the desired supporting action of the foot sole without traumas and ensuring complete offloading of the ulcer.

Therefore, the insert 10 ensures offloading of the ulcer to the same degree as that which is achieved by the known offloading systems.

Advantageously, the insert may be made of a material with a double density, in which the portion with a lower density, which is therefore softer, is intended to be arranged close to the ulcer, and the portion with a greater density, which is therefore harder, is intended to be arranged further away from the wound.

The insert 10 may be made by means of cast or injection moulding inside a suitably shaped mould.

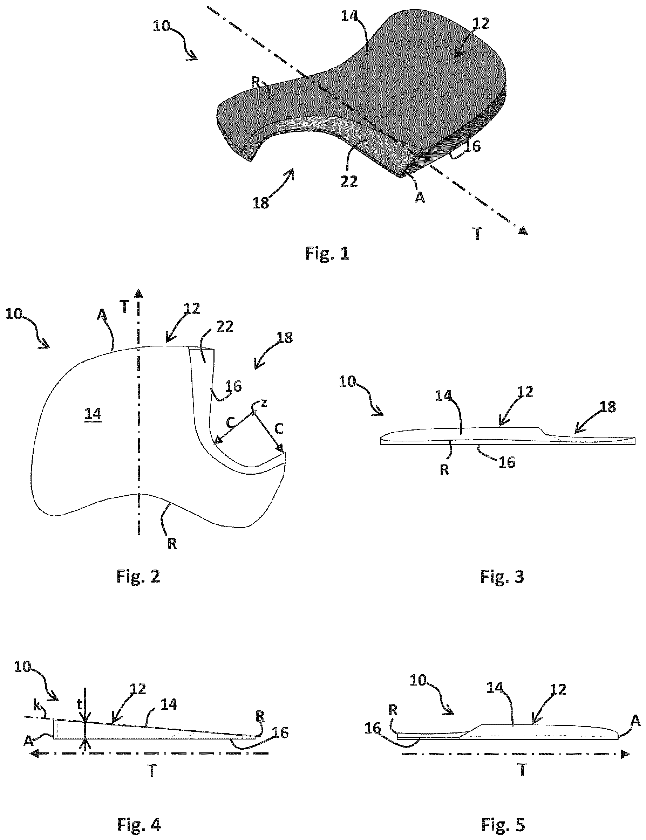

As is clearly visible in the attached figures (see for example Figs. 4, 8 and 12), the thickness t of the body 12 is greater at a front end A than at a rear end R of the insert 10. Preferably, the thickness t increases gradually from the rear end R towards the front end A of the insert 10 along a longitudinal axis T.

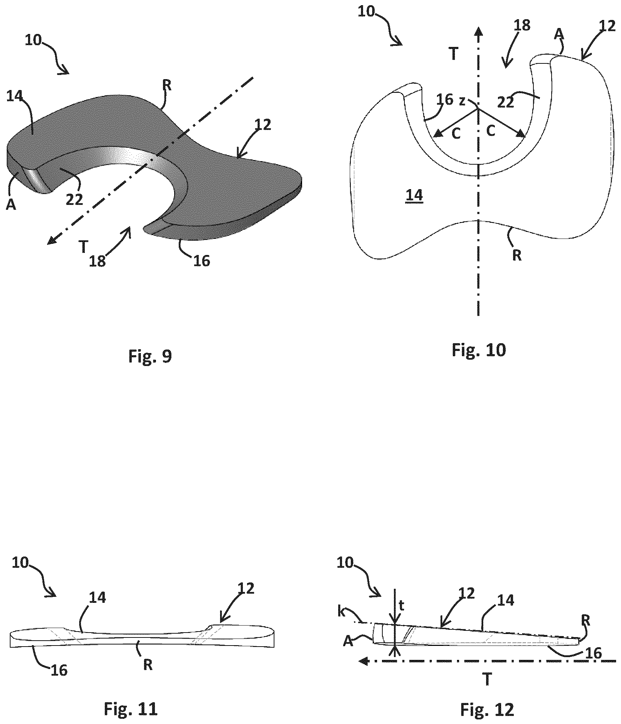

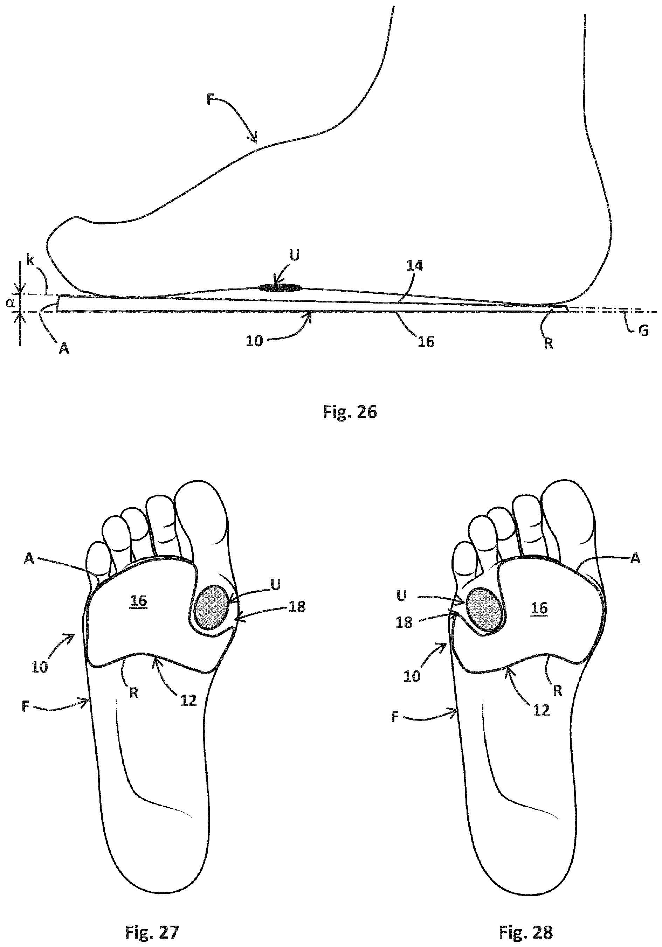

In this way the upper surface of the insert 10 is inclined along a direction k with respect to the lower surface 16 (see for example Figures 4 and 12). Advantageously, this solution may also be adopted in the case where the insert 10 extends along the entire length of the foot (see for example Figure 26).

In this case, the upper surface 14 may have an inclination k which defines with reference to the lower surface 16 an angle α within a range of between 2° and 8°.

This inclination k allows the ulcer area to be offloaded further by transferring part of the weight, and the associated pressures, from the front portion of the foot to the hindfoot portion.

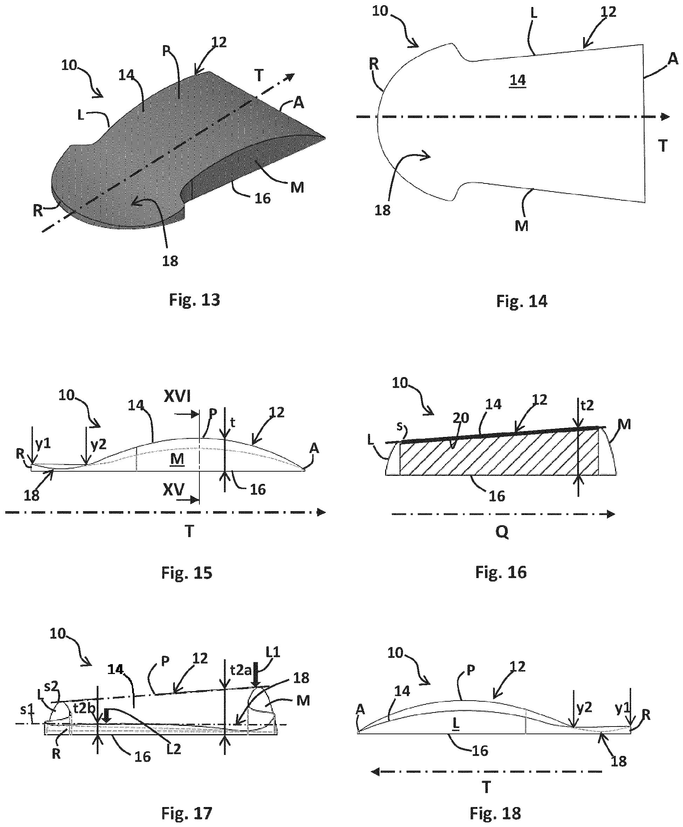

In an alternative embodiment (see for example Figure 15), the thickness t1 of the body 12 of the insert 10 gradually increases from a rear end R along the longitudinal axis T, and then decreases gradually at a front end A of the insert 10.

In this case, the upper surface 14 of the insert 10 defines, in fact, a protuberance P with respect to the lower surface 16.

Advantageously, as shown for example in Figure 16, which shows an insert 10 configured to support the hindfoot of a left foot, the body 12 of the insert 10 may have a transverse thickness t2 which varies gradually also along a direction Q transverse to the longitudinal axis T.

As is clearly visible in Figure 16, the transverse thickness t2 of the insert 10 may decrease in the direction from a medial zone M towards a lateral zone L of the insert 10. In this way, the upper surface 14 of the body 12 of the insert 10 may be inclined along a transverse direction s with respect to the lower surface 16, so as to follow the anatomy of the foot which on average is higher in the medial zone.

Advantageously, still with reference to the embodiment shown in Figures 13-18 and in particular to Figure 17, the transverse thickness t2a, t2b of the body 12 of the insert 10 calculated along two different transverse sections may gradually vary in a different manner between the medial zone M and the lateral zone L such that the upper surface 14 of the insert 10 may have two different transverse inclinations with respect to the lower surface 16, said inclinations being incident to each other. In Figure 17, for example a first transverse inclination s1 and a second transverse inclination s2 are visible. Along the transverse inclination s2, the medial zone M has transverse thicknesses t2a greater than the lateral zone L, and along the transverse inclination s1, the lateral zone L has transverse thicknesses t2b greater than the medial zone M.

The resultant upper surface 14 is in fact an undulating surface which has two different transverse inclinations.

As already mentioned, the upper surface 14 is configured to support a given portion of the user's foot.

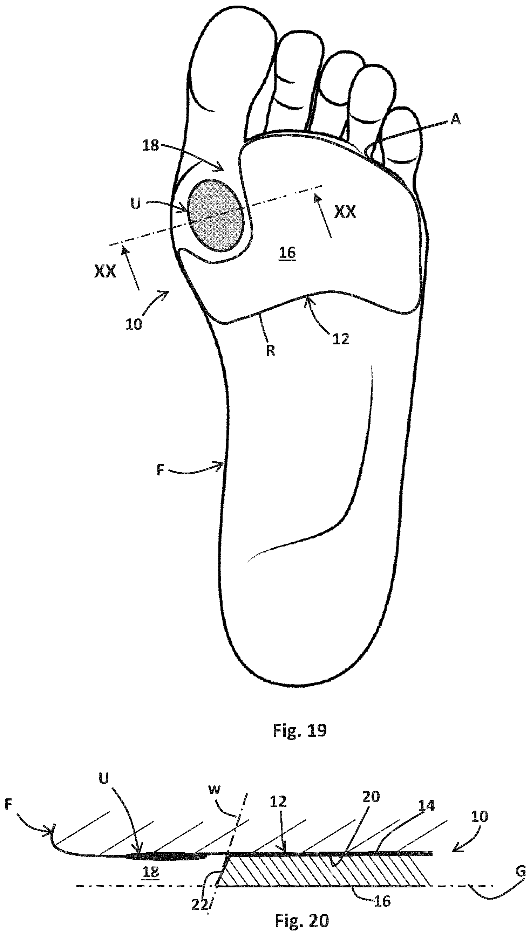

As shown schematically in Figures 1, 6, 9 and in Figures 19, 21, 22, the upper surface 14 of the body 12 may be shaped anatomically so as to follow the profile of the forefoot portion of the foot F of the user. The aforementioned figures show, by way of example, inserts 10 intended to be applied on the plantar surface of left feet (see for example Figures 19, 21 and 22). The inserts for the right feet have a design which is a mirror image of the inserts for the left feet (see for example Figures 27 and 28).

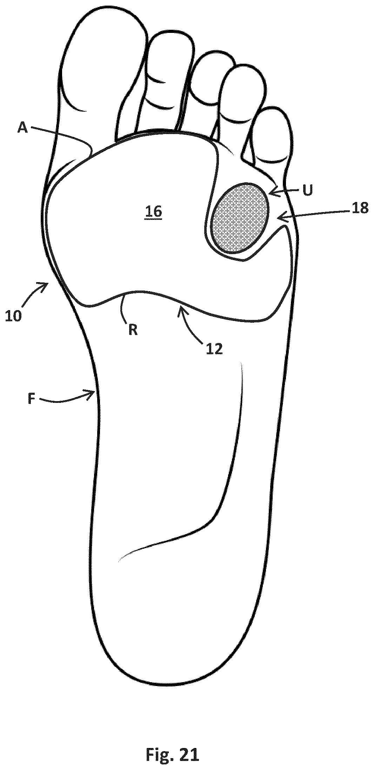

In detail, Figure 27 shows the same insert shown in Figures 19, but applied to a right foot. Similarly, Figure 28 shows the same insert shown in Figure 21, but applied to a right foot.

Alternatively (see for example Figures 13 and 23-25), the upper surface 14 of the body 12 is shaped anatomically to follow the profile of the hindfoot portion of the foot F of the user.

In this case also, the aforementioned figures schematically show, by way of example, inserts intended to be applied on the plantar surface of left feet. The inserts for right feet have a design which is a mirror image of these inserts.

Advantageously, an adhesive layer 20 may be applied onto at least part of the upper surface 14 of the body 12 (see Figures 16 and 20). Preferably, the adhesive layer 20 is applied over the entire upper surface 14.

Said adhesive layer 20 allows the insert 10 to adhere to the skin of the foot, preventing the insert 10 from moving during use.

In a preferred embodiment, said adhesive layer 20 consists of a bi-adhesive layer applied onto the upper surface 14.

Said adhesive 20 is configured to be placed in direct contact with the portion of the foot F of the user onto which the insert 10 is applied. Advantageously, both the body 12 and the adhesive layer 20 are made of dermal compatible materials.

In accordance with the invention, the insert 10 is characterized in that the body 12 comprises a concavity 18 configured to be arranged facing a plantar skin lesion U, such as an ulcer.

With reference firstly to Figures 1-12, said concavity 18 may be arranged along a front portion of the insert 10.

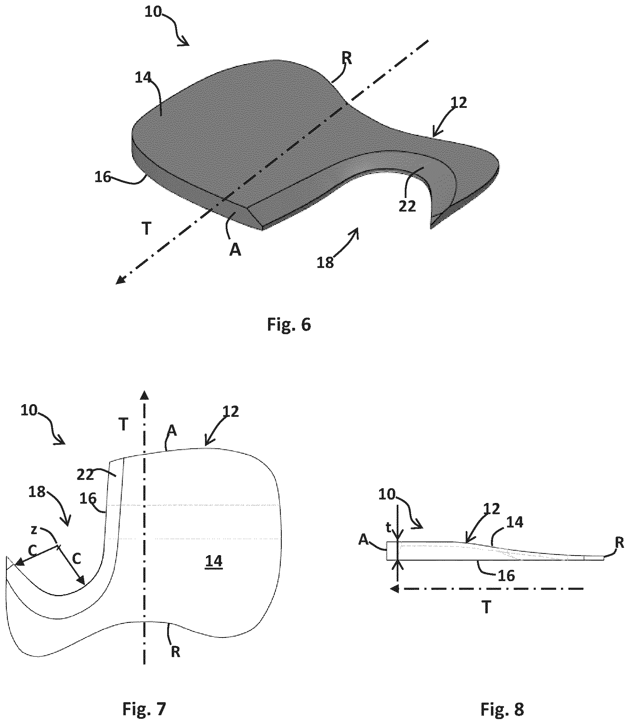

In particular, the concavity may be arranged along a medial front portion (see Figure 2) or along a lateral front portion (see Figure 7) or along a central front portion (see Figure 10).

As will be described again below, the concavity provided along the medial front portion allows offloading of ulcers positioned below the head of the 1^{st} metatarsus.

The concavity provided along the lateral front portion allows offloading of ulcers positioned below the head of the 5^{th} metatarsus.

The concavity provided along the central front portion allows offloading of ulcers positioned below the heads of the central metatarsi.

Advantageously, as already mentioned, the inserts intended to be applied to right feet have a design which is the mirror image of the inserts intended to be applied to left feet, so as to be able to follow in any case the anatomy of the foot.

Alternatively, with reference to Figures 13-18, said concavity 18 may be arranged along a rear portion of the insert 10.

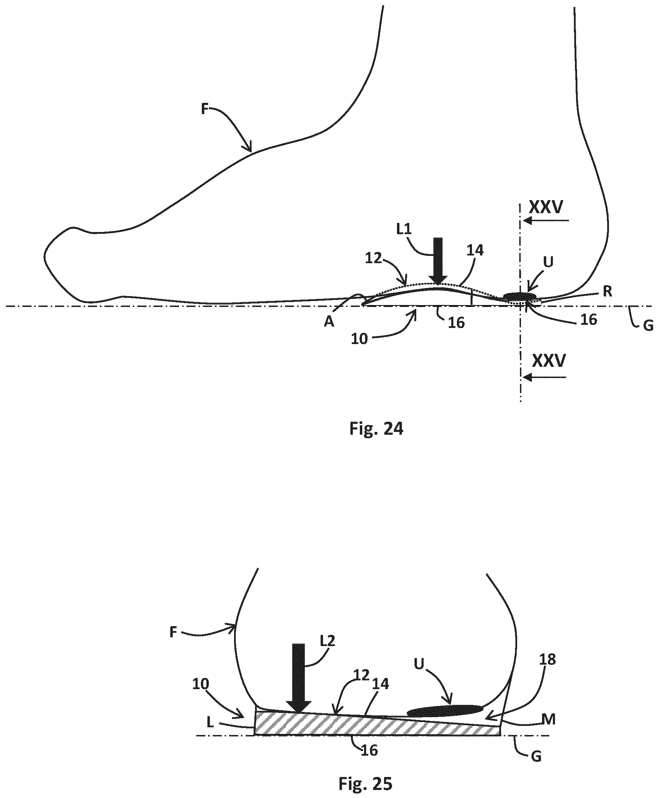

Preferably, in said embodiment, the concavity 18 is arranged along a medial rear portion of the insert 10 (see Figure 25).

Advantageously, with reference to Figures 1-12, the concavity 18 may cross the entire thickness t of the body 12, so as to be at least partially delimited by a lateral edge 22 which joins the upper surface 14 together with the lower surface 16.

Advantageously, as is clearly visible in Figure 20, said lateral edge 22 may be inclined with respect to the upper surface 14 along a direction w so as to define an inclined surface which causes a gradual narrowing of the concavity 18 in the direction from the upper surface 14 towards the lower surface 16.

In this way, on the one hand it is ensured that the lower surface 16, where the concavity 18 has smaller dimensions, ensures suitable supporting of the foot. On the other hand, it is ensured that there is less risk of the upper surface 14, where the concavity 18 has larger dimensions, coming into contact with the skin lesion U.

Advantageously, in the case where the concavity 18 passes through the body 12 of the insert 10, said concavity 18 may have a perimetral profile shaped substantially in the form of an L (see Figure 2) or in the form of a J (see Figure 7). In this embodiment, the concavity is substantially open on both sides, at the front and on the sides, and is arranged on a medial or lateral portion of the body 12.

Alternatively, the concavity may have a perimetral profile shaped substantially in the form of a U (see Figure 10). In this case, the concavity is substantially open on one side only, at the front, and is arranged on a central portion of the body 12.

Still with reference to Figures 1-12, the concavity 18 may have an oblong shape. Advantageously, the rear section of the concavity 18 may have a substantially circular shape with a radius C (see Figures 2, 7 and 10). Said radius C is centred around a centre of curvature z.

In an alternative embodiment (see Figures 13-18), the concavity 18 consists of a depression formed in the upper surface 14. In this case, therefore, the concavity 18 does not pass through nor extends across the entire thickness 12 of the insert 10.

In this embodiment, the concavity 18 is a dip defined between two peaks y1, y2 formed on the upper surface 14 of the body 12 of the insert 10. Said peaks y1, y2 are visible in Figures 15 and 18.

In other words, if a hypothetical straight line is traced so as to join together said peaks y1, y2, it is possible to define a depression in the upper surface 14 of the insert 10.

In this embodiment also, therefore, the ulcer U advantageously does not come into direct contact with the insert 10 since the foot may rest on the two peaks y1, y2.

Preferably, in this embodiment, the insert may be provided with a protuberance P and have transverse thicknesses t2a, t2b which are not uniform, as shown in Figure 17.

Said configuration is specifically designed so that the hindfoot may rest on, and therefore be supported by, the points L1 and L2 defined on the upper surface 14 of the insert 10 (see Figures 17, 23-25) so as to ensure that the ulcer U does not come into direct contact with the insert or with a support surface, for example the ground or a part of the shoe worn by the user.

As already mentioned and as schematically shown in Figures 19-22, the body 12 of the insert 10 may be shaped so as to follow the profile of the forefoot of the user's foot.

In such an embodiment, in the case where the concavity 18 is arranged in the medial front portion, the insert may advantageously be used to treat skin lesions positioned along the 1^{st} metatarsal radius (see for example Figure 19).

In the case where the concavity 18 is provided in the lateral front portion, the insert 10 may advantageously be used to treat skin lesions which have formed along the 5^{th} metatarsal radius (see for example Figure 21).

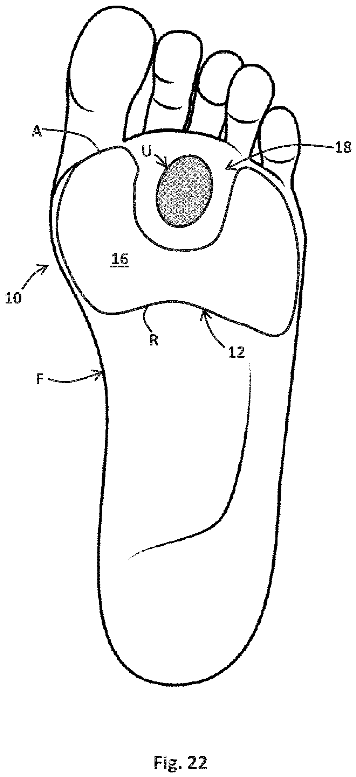

In the case where the concavity 18 is provided in the central front portion, the insert may advantageously be used to treat skin lesions formed along the 3^{rd} metatarsal radius (see for example Figure 22).

By means of the three inserts described above it is possible to treat nearly all the ulcers U present on the forefoot in diabetic patients.

As clearly shown in Figure 20, the arrangement of the concavity 18 allows an insert 10 to be obtained such that it supports the patient's foot when it rests on a hypothetical support surface G, without the user's weight bearing also on the ulcer U.

In this way, the patient's movements are not hampered.

Furthermore, owing to the provision of a gradually increasing thickness between the rear end and the front end of the insert body, the latter is able to keep the foot in dorsiflexion. The pressure exerted by the weight of the patient in the vicinity of the ulcer is therefore reduced. This advantageously ensures that the zone surrounding the ulcer is not subject to high compressive forces and therefore reduces the risk that the ulcer may expand, with the resultant advantages.

These advantages are also obtained with the insert of Figure 26 intended to extend underneath the whole of the foot sole.

Moreover, the possibility of fixing the insert directly to the foot sole by means of the adhesive 20 ensures that the insert remains in the correct position during use, further reducing the risk of it interfering with the natural movements of the user's foot.

The same comments are also applicable to the embodiment of the insert shown in Figures 13-18.

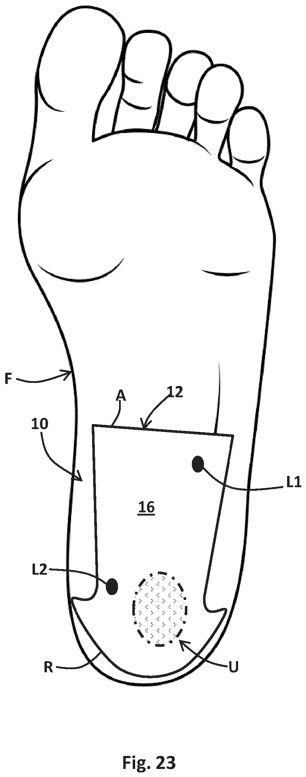

The body 12 of the insert 10 is shaped to follow the profile of a hindfoot portion of the user's foot (see Figure 23).

In this embodiment, as mentioned, the concavity 18 is a dip formed in the upper surface 14 of the insert 10 between two peaks y1 and y2.

The insert is provided with a protuberance P and is shaped so that the foot may rest on two points L1, L2 which are spaced from each other and formed on the upper surface 14, in a medial zone M and lateral zone L, respectively, so as to ensure that the concavity 18 facing the ulcer U does not come into contact with the foot.

In this case also, therefore, even though a through-concavity has not been provided, the body of the insert does not come into direct contact with the ulcer and therefore allows the patient to move freely (see Figures 24 and 25).

This insert advantageously allows treatment of almost all the ulcers which occur in the heel zone. Such ulcers occur in the central zone of the heel, towards the medial part thereof.

The provision of the concavity 18 as described above, together with the provision of a protuberance P with greater transverse thicknesses t2 in the medial zone, ensure that the weight of the body does not bear on the part of the foot where the ulcer U is present.

As already mentioned, the present invention also relates to a kit comprising a plurality of different disposable inserts 10, each insert 10 of said plurality being made as described above.

The kit may advantageously comprise at least two inserts of each type described above, one for the right foot and one for the left foot, so as to be able to treat ulcers present in the forefoot zone and ulcers present in the hindfoot zone.

Furthermore, in view of the fact that diabetic ulcers occur in adult patients having a foot size which varies from a size 35 to a size 48, measured using the French system, with an increase of 6.66 mm lengthwise and 1.5 mm widthwise, the kit may comprise a plurality of disposable inserts 10 of the same type and having different sizes.

For example, in the case where the insert is intended to be applied to the foot of an adult man, the kit may comprise a first insert configured to be applied to feet with a size of between 39 and 41, a second insert configured to be applied to feet with a size of between 42 and 44, and a third insert configured to be applied to a feet with a size of between 45 and 48.

Similar considerations obviously also apply in the case where the insert is intended to be applied to a woman's foot, where the foot sizes generally vary from 35 to 41 according to the French system.

In this case also, the kit may comprise inserts designed to be applied to women's feet of different sizes.

Advantageously, the inserts in the kit will be contained inside sterile packaging, divided up according to the size and type associated with the location of the ulcer.

In view of the fact that the medication used for the ulcers, according to the guideline protocol, must be changed every 3-5 days, advantageously the professional operators (doctors, podiatrists and nurses) will have available a sterile kit for each ulcer location, to be used together with the medication, thus improving its healing effect, owing to the offloading ensured by the insert 10.

This thus results in the provision of an insert which is shaped to suit the specific needs of the patient, which may be easily applied and which, since it is disposable, has a low cost, while ensuring adequate support for the patient's foot.

The insert will be preferably replaced every 2 or 3 days or at least whenever medication is applied.

The advantages which may be achieved with the insert and the kit according to the invention are therefore clear from the above description. The insert according to the invention is, in fact, practical and easy to use also by persons who do not require specialized training.

Moreover the insert may be produced at a low cost, for example, by means of moulding, without this affecting its dimensional precision, which is indispensable for ensuring adequate treatment of plantar ulcers.

Finally, the possibility of providing the various inserts in the form of kits, simplifies the use thereof by the patients.

The person skilled in the art, in order to satisfy specific needs, may make modifications to the embodiments of the insert described above and and/or replace the elements described with equivalent elements, without thereby departing from the scope of the attached claims.

## Claims

1. Disposable insert (10) for the treatment of plantar skin lesions (U), comprising a body (12) made of a polymer material and having a thickness (t) variable along a longitudinal axis (T) thereof; said body (12) comprising an upper surface (14), configured to support a given portion of a foot (F) of a user, and an opposite lower surface (16);
**characterized in that** said body (12) comprises a concavity (18) which is configured to be arranged facing, when the disposable insert (10) is being used, a plantar skin lesion (U) of the foot (F) of the user, so as to prevent said plantar skin lesion (U) from coming into contact with the body (12) of the insert (10) or with a support surface.

2. Disposable insert (10) according to Claim 1, **characterized in that** said concavity (18) crosses the entire thickness (t) of the body (12) so as to be at least partially delimited by a lateral edge (22) which joins the upper surface (14) together with the lower surface (16).

3. Disposable insert (10) according to Claim 2, **characterized in that** said lateral edge (22) is inclined with respect to the upper surface (14) along a direction (w), so as to define an inclined surface which causes a gradual narrowing of the concavity (18) in the direction from the upper surface (14) towards the lower surface (16).

4. Disposable insert (10) according to any one of the preceding claims, **characterized in that** the upper surface (14) of the body (12) is shaped anatomically so as to follow the profile of a forefoot portion of the foot (F) of the user or to follow the profile of a hindfoot portion of the foot (F) of the user.

5. Disposable insert (10) according to any one of the preceding claims, **characterized in that** said concavity (18) is provided along a front portion of the insert (10).

6. Disposable insert (10) according to Claim 5, **characterized in that** said concavity (18) is arranged along a medial front portion or along a lateral front portion or along a central front portion.

7. Disposable insert (10) according to Claim 1, **characterized in that** said concavity (18) consists of a depression formed in the upper surface (14) of the body (12) of the insert (10).

8. Disposable insert (10) according to Claim 7, **characterized in that** said concavity (18) is a dip defined between two peaks (y1, y2) formed on the upper surface (14) of the body (12) of the insert (10).

9. Disposable insert (10) according to Claim 7, **characterized in that** the upper surface (14) of the body (12) is shaped anatomically so as to follow the profile of a hindfoot portion of the user's foot, said concavity (18) being arranged along a rear portion of the insert (10).

10. Disposable insert (10) according to Claim 2, **characterized in that** said concavity (18) has a perimetral profile shaped substantially in the form of an L or a J or a U.

11. Disposable insert (10) according to any one of the preceding claims, **characterized in that** the body (12) has a transverse thickness (t2; t2a, t2b) which varies gradually along a direction (Q) transverse to the longitudinal axis (T).

12. Disposable insert (10) according to Claim 11, **characterized in that** the transverse thickness (t2a, t2b) of the body (12) of the insert (10) calculated along two separate transverse sections varies gradually in a different manner between a medial zone (M) and a lateral zone (L) such that the upper surface (14) of the insert (10) has two different transverse inclinations with respect to the lower surface (16).

13. Disposable insert (10) according to Claim 1, **characterized in that** the thickness (t) of the body (12) gradually increases from a rear end (R) towards a front end (A) of the insert (10) along a longitudinal axis (T).

14. Disposable insert (10) according to Claim 1, **characterized in that** the thickness (t) of the body (12) gradually increases from a rear end (R) along the longitudinal axis (T) and then decreases gradually at a front end (A) of the insert (10); the upper surface (14) defining a protuberance (P) with respect to the lower surface (16).

15. Disposable insert (10) according to any one of the preceding claims, **characterized in that** an adhesive layer (20) is applied onto at least part of the upper surface (14); said adhesive layer (20) being configured to be placed in direct contact with the portion of the foot (F) of the user onto which the insert (10) is applied.

16. Disposable insert (10) according to any one of the preceding claims, **characterized in that** it has a hardness measured on the Shore A scale of between 20 and 50.

17. Disposable insert (10) according to any one of the preceding claims, **characterized in that** the body (12) is made of a closed-cell polymer material with a density of between 150 and 450 Kg/m³.

18. Kit comprising a plurality of different disposable inserts (10) for the treatment of plantar skin lesions (U), said kit being **characterized in that** each disposable insert (10) of said plurality is made in accordance with any one of Claims 1 to 17.

19. Kit according to Claim 18, **characterized in that** it comprises a plurality of disposable inserts (10) of the same type and of different sizes.

20. Kit according to Claim 19, **characterized in that** said disposable inserts (10) are contained inside sterile packaging, divided up according to the size and type associated with the location of the plantar skin lesion (U) in the foot (F) of the user.
